# EUROPEAN PATENT SPECIFICATION

(11) **EP 1 231 972 B1**
(45) Date of publication and mention of the grant of the patent: **18.01.2006**
(21) Application number: 00983678.4
(22) Date of filing: 24.11.2000
(51) Int. Cl.: A61M 25/00

(54) **ELLIPTICAL WIRE-REINFORCED CATHETER**
VERSTÄRKTER KATHETER MIT ELLIPTISCHEM DRAHT
CATHETER RENFORCE PAR UN FIL A SECTION ELLIPTIQUE

(30) Priority: 26.11.1999 US 167613 P
(43) Date of publication of application: 21.08.2002
(73) Proprietor: MICRO THERAPEUTICS, INC., Irvine, CA 92618 (US)
(72) Inventor: KIM, James, H., Garden Grove, CA 92840 (US); STRAUSS, Brian, M., Trabuco Canyon, CA 92692 (US); SLEE, Earl, H., Laguna Niguel, CA 92677 (US)
(74) Representative: Poulin, Gérard
(86) International application number: PCT/US2000/030157
(87) International publication number: WO 2001/037918

(56) References cited:
- US-A- 5 704 926
- US-A- 5 713 867
- US-A- 5 951 539
- US-A- 5 964 971

## Description

### 1. Field of the Invention

The invention relates to wire-reinforced catheters used for detection and/or delivery of material to and from remote locations within the body of a patient. See for example US-A-5964971, which describes the closest prior art.

### 2. Description of Related Art

Wire-reinforced catheters are well known in the art. Generally, these consist of an elongated, flexible tubular body defining a central lumen extending from one end of the body to the other end. The lumen communicates with the exterior of the body at the ends. In this manner, when a distal end of the catheter is implanted in the body of a patient, the lumen provides a conduit for delivery of material to or from the body, or for transfer of sensor information from within the interior of the body.

Structurally, the tubular body of the catheter is formed of a polymeric or other material, typically formed in layers. One arrangement, of particular interest here, contemplates a structure in which an inner layer is surrounded by a wound reinforcing wire. Atop this reinforcing wire is overlaid an outer layer, such that the reinforcing wire is sandwiched between the inner and outer layers. The winding pitch of the reinforcing wire can be varied along the length of the catheter to achieve a desired flexibility profile. One or more wires can be used, spirally wound in the same or opposite directions, extending partially or completely along the length of the catheter. Multiple, counter-woven strands thus used can be considered as forming a reinforcing wire mesh between the inner and outer layers of the catheter. In cross section, the wires deployed in the prior art are either round or rectangular.

One prior art device is the subject of U.S. Pat. No. 3,924,632 to Cook, disclosing a catheter 10 having inner (30) and outer (35) layers of plastic material such as polyethylene. A mesh of woven fiber glass bands 40, 41, 42, 43, 48, 49, 50 and 51 is disposed between these layers, the mesh comprising counter-rotated strands spirally wound around the exterior of inner layer 30. The strands are of 0.01 mm thickness. Layers 30 and 35 are heat bound to each other through the interstices of the fiber glass mesh. To provide maximal flexibility at the tip of catheter 10, the distalmost portion comprises a meshless tube 52 bonded to the end.

U.S. Patent No. 4,516,972 to Samson teaches the use of a ribbon reinforcement layer 16 disposed between an inner liner 12 and an outer layer 26 of a catheter 11. The ribbon is wound around inner liner 12 at varying pitches in order to achieve a specific flexibility profile in which proximal end portion 12a, intermediate portion 12b, and distal portion 12c each exhibit different flexibility. The ribbon is omitted altogether from a tip portion of the catheter 11 for maximum flexibility. One or two layers (17, 18) of ribbon can be used, counter rotated, with the second layer overlying the first layer. The ribbon material is preferably Kevlar.

U.S. Patent No. 4,425,919 to Alston, Jr. et al. discloses a catheter 10 which is provided with a flat wire braid 14 disposed between an inner layer 12 and an outer layer 16. The inner layer 12 is made from a stretched, pre-oriented polyvinylidene fluoride or nylon 12. Outer layer 16 materials can be polyolefin polymers or ethyl vinyl acetate or polyurethane, adapted for sterilization by radiation. A flexible tip is provided by extending the outer layer beyond the inner layer and wire braid, so that the outer layer alone forms the tip and results in a more flexible structure.

U.S. Patent No. 5,037,404 to Gold, et al. shows a catheter 10 having an inner layer 12 made from polyethylene, nylon, PVC, polyurethane or silicon rubber. Layer 12 is surrounded by a helically wound wire sheath 16 having a pair of counter rotated wires 18, 20 whose relative angle (i.e., pitch) can be varied to achieve desired torsional and longitudinal stiffness characteristics in different sections. The number of sections may be two or more, depending on the application, and the possibility of three sections is discussed. The wires 18, 20 can be braided, or they can be configured such that one overlies the other. Any desired wire cross-section may be used, and possible wire materials include stainless steel, nylon, and memory alloys such as Nitinol.

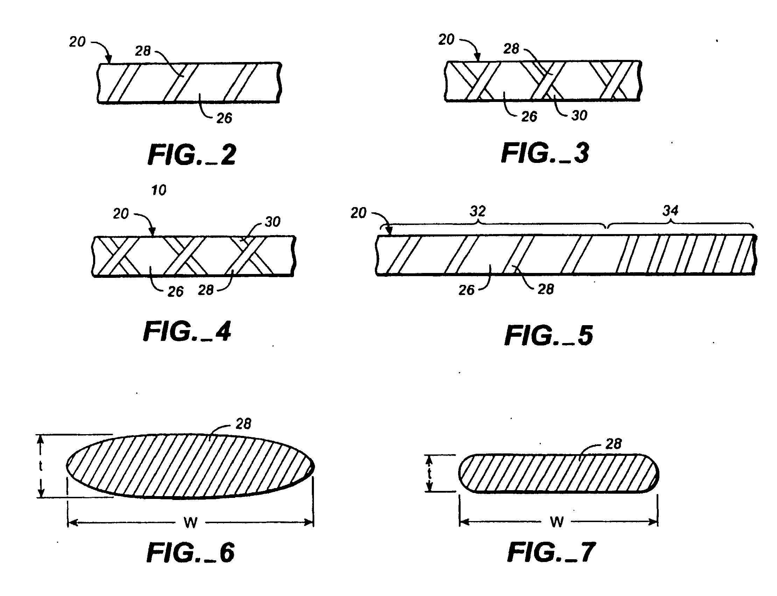

U.S. Patent No. 5,069,674 to Fearnot, et al. shows the use of a spirally wound wire 105 to reinforce a catheter 100 comprised of an outer sheath 104 and an inner tube 401 (see Fig. 4). The coils of the wire 105 are loosely wound at the distal end of the catheter in order to achieve a more flexible structure at that end. The material of the wire 105 is hardened stainless steel "or other metals or alloys", while the material of the outer sheath 104 can be polyamide, fluoropolymers, TEFLON™ or other copolymer plastics.

U.S. Patent No. 5,279,596 to Castaneda, et al. shows a catheter 10 having a distal portion 22 which is more flexible than a proximal portion 14. In both portions a support wire is embedded. Wire 28 of the distal portion is preferably of rectangular cross-section and is made of stainless steel. Structurally, wire 28 coaxially surrounds an inner layer 26 and is embedded in an overlaying outer layer 24 (see Fig. 4). Inner layer 26 is formed of PTFE, while outer layer 24 is formed of nylon. The relative difference in flexibility between distal portion 22 and proximal portion 14 is achieved using appropriate materials selection, particularly, by selecting for the outer layer of the distal portion a material with a lower Shore D durometer rating (a rating of 40 is mentioned in col. 3, 11. 63-64) than that of the outer portion of the proximal portion.

U.S. Patent No. 5,176,660 to Truckai, et al., discusses the Gold, et al. patent (see above) and its teachings are specifically held out as an extension of the concepts of the Gold, et al. patent. In Truckai, a catheter 10 is provided with an inner layer 12 made of polyethylene, nylon, PVC, polyurethane, or silicon rubber. A tubular braided wire sheath 16 is formed over inner layer 12. The braided wire sheath 16 comprises a pair of counter rotating helical strands 18,20 of flat wire made from spring steel. Nitinol is also discussed, in col. 4, 1. 40. Over the braided wire sheath 16 and bonded with the inner layer 12 via the interstices of the sheath is an outer layer 22, made of the same material as the inner layer, as seen from Fig. 5. The angle strands 18 and 20 make with each other can be varied in different segments of the catheter 10 to achieve varying physical characteristics, as discussed in col. 3, 11. 17-19 and col. 4, 11. 21-23. These characteristics include stiffness/flexibility.

U.S. Patents Nos. 5,454,795, 5,695,483 and 5,876,386 to Satnson describe a catheter in which one or more stiffener ribbons (274) are used to reinforce the tubular body (270) of the catheter and control flexibility. The stiffener ribbons are selectively applied in varying pitches and turn directions ("handedness") in order to tailor the catheter flexibility profile to specific applications. The catheter is constructed to have the stiffener ribbons sandwiched between inner and outer tubular polymer layers, referred to respectively as an inner tubular liner (272) and outer tubular cover (276). The ribbons are described as rectangular in cross section, having dimensions of between 0.018 mm and 0.038 mm in thickness and between 0.063 mm and 0.20 mm in width. Alternatively, depending on the ribbon material, thickness and width dimensions of 0.01 mm and 0.025 mm, respectively, are mentioned. The tubular polymer layers comprise compatible materials designed to adhere to each other with or without the aid of a suitable adhesive.

The above-described prior art references are herein incorporated by reference in their entirety.

The prior art has been found to suffer from shortcomings due to the cross-sectional shape of the reinforcing wires used. Specifically, the use of round wire fails to provide the desired stiffness control, and requires wires whose thickness is dimensionally incompatible with the design and size requirements for many applications. Rectangular cross section wires, on the other hand, have sharp edges which can cause damage to the tubular body of the catheter during manufacture and/or use.

### BRIEF SUMMARY OF THE INVENTION

To overcome the deficiencies of the prior art, the present invention provides a catheter having a reinforcing wire whose cross sectional shape is oval or elliptical. In this manner, the potentially problematic sharp edges of the rectangular reinforcing wire of the prior art are avoided. Moreover, the wire is not round in cross section, thus providing improved functional and dimensional performance over the known prior art.

In accordance with the present invention, a catheter having a substantially tubular body in which a main lumen is formed is provided with a helically wound reinforcing wire in at least a portion thereof. This portion may be the distalmost, implantable portion of the catheter, or it may be a different portion or the entirety of the catheter. The tubular body comprises inner and outer layers of identical or different materials between which the reinforcing wire is helically wound. The reinforcing wire is selected to have an oval or elliptical cross section, thereby reducing the potential for damage to the inner and outer layers of the catheter due to the presence of sharp edges. Additionally, the oval or elliptical cross sectional shape affords the advantages of a smaller profile and improved performance, compared to bulkier and less suitable conventional round wire devices.

The winding configuration selected in the catheter of the present invention is a function of the desired catheter flexibility profile, with a higher winding pitch being used to impart greater flexibility. Additionally, flexibility can be controlled through judicious selection of the materials for the reinforcing wire and the inner and outer layers.

### BRIEF DESCRIPTION OF THE SEVERAL VIEWS OF THE DRAWING(S)

Many advantages of the present invention will be apparent to those skilled in the art with a reading of this specification in conjunction with the attached drawings, wherein like reference numerals are applied to like elements and wherein:
FIG. 1 is a diagrammatical view of a catheter in accordance with the invention;
FIG. 1A is a sectional view of a segment of the catheter of FIG. 1;
FIG. 2 is a schematic view showing a first reinforcing wire winding configuration;
FIG. 2A is a sectional view of an alternative embodiment of a segment of the catheter of FIG. 1;
FIG. 3 is a schematic view showing a second reinforcing wire winding configuration;
FIG. 4 is a schematic view showing a third reinforcing wire winding configuration;
FIG. 5 is a schematic view showing a fourth reinforcing wire winding configuration;
FIG. 6 is a cross-sectional view depicting a first exemplary reinforcing wire shape in accordance with the invention; and
FIG. 7 is a cross-sectional view depicting a second exemplary reinforcing wire shape in accordance with the invention.

### DETAILED DESCRIPTION OF THE INVENTION

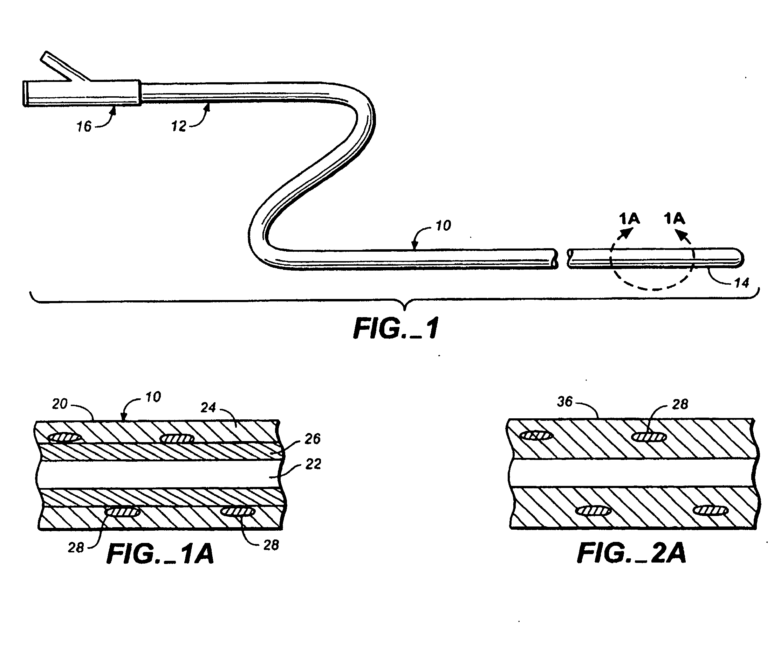

FIG. 1 shows diagrammatically a catheter 10 in accordance with the present invention. Catheter 10 is an elongate, generally tubular structure having a proximal portion 12 and a distal portion 14. A fitting, designated generally as 16, is disposed at proximal portion 12 and serves to interface the catheter 10 with various support and delivery devices, depending on the contemplated application of the catheter. Catheter 10 in operation is introduced into the body of a patient (not shown) such that the distal portion 14 is guided, using for instance a guidewire (not shown), to a target site within the body for performance of a needed procedure, such as localized delivery of medicament to the site. Proximal portion 12 remains exterior to the patient and provides access, via a main lumen (as described below), to the site within the patient's body.

FIG. 1A is a partial sectional view of a segment 20 of catheter 10 in accordance with the invention. Segment 20 is an exemplary portion of the catheter 10, such that the structure of segment 20 as described can be specific to a prescribed portion of the catheter, such as distal portion 14, or can be illustrative of the construction of the entire length of the catheter. Segment 20 comprises a main lumen 22 extending longitudinally along the segment length and defined by an inner layer 26 and an outer layer 24. Main lumen 22 extends substantially the entire length of catheter 10, from proximal portion 12 to distal portion 14, and is in communication with the exterior of the catheter at least at these portions and/or in their vicinity. Lumen 22 serves to convey materials between proximal portion 12 and distal portion 14 of catheter 10, which materials include but are not limited to nutrition, medicaments, contrast media, liquid embolizing agents, blood, guidewires and other devices such as sensors and sensor information-carrying conductors. Layers 24 and 26 are generally tubular in shape, resulting in a generally tubular segment 20 and catheter 10.

Numeral 28 represents a reinforcing wire overlying inner layer 26 and covered by outer layer 24. Wire 28 is preferably disposed in a helical pattern, illustrated diagrammatically in FIG. 2. Other patterns are also possible, as illustrated in FIGS. 3-5. In FIG. 3, two wire strands are used, one overlying the other. Specifically, in FIG. 3, wire 28 is wrapped around inner layer 26 and around a second, inner wire 30. Wires 28 and 30 can be wound in the opposite directions, as depicted in FIG. 3, or they can be wound in the same direction and offset such that one wire is disposed between the other wire to provide a denser weave pattern―that is, a higher pitch (not shown). In FIG. 4, the two wires 28 and 30 are wound in opposite directions and interwoven, to effectively form a reinforcing mesh over inner layer 26. In FIG. 5, segment 20 is shown as divided into two segments, 32 and 34, each having the wire 28 wound at a different pitch over inner layer 26. Different pitches are used to selectively control flexibility of different portions of catheter 10. Moreover, in some constructions combinations of the above patterns can be used, in different segments or within the same segment, depending on the desired characteristics.

It is also contemplated that the catheter 10 can be constructed of a single layer 36. In such a construction, reinforcing wire 28 is embedded in layer 36 using standard manufacturing techniques. FIG. 2A depicts such an arrangement.

An important feature of the present invention is the cross-sectional shape of the wires 28 and 30. In order to eliminate sharp edges while reducing the profile of the wire, an elliptical or oval shape is selected. FIGS. 6 and 7 depict such shapes. It will appreciated that adherence to strict definitions of elliptical-that is, in the conic curve sense-is not intended, and some deviation is contemplated. It will also be appreciated that the term "wire" is not intended to designate an electrically conductive material or a metallic material, although the latter construction is in fact preferred. For purposes of completion, however, it will appreciated that a suitable polymeric material or other non-metallic materials fall within the purview of the present invention.

Dimensionally, it is contemplated that the wire have a thickness t of between about 0.01 mm to about 0.1 mm and a width w of between about 0.038 mm to about 0.38 mm Other dimensional combinations for the wire can be a thickness of about 0.01 mm and a width of about 0.038 mm a thickness of about 0.03 mm and a width of about 0.08 mm a thickness of about 0.05 mm and a width of about 0.20 mm or a thickness of about 0.1 mm and a width of about 0.38 mm. Other dimensional combinations are also contemplated and it is not intended that the invention be limited to those enumerated above.

Catheter 10 is constructed from materials which are well known in the art and which are governed by the particular application with a view to for example the flexibility and torquability requirements, along with the particular procedure to be performed on the patient, patient size and condition, and the materials to be delivered to the target site in the patient's body. It is preferred that the catheter be constructed to meet certain minimal physical criteria. Specifically, for many applications it is preferred that the distal portion of the catheter adhere to a critical bend diameter constraint of no more than about 1.50 mm and exhibit a lateral stiffness of greater than about 7.3°/cm-g deflection measured by a Tinius-Olsen Stiffness Tester at 20°-30° of deflection.

The above are exemplary modes of carrying out the invention and are not intended to be limiting. It will be apparent to those of ordinary skill in the art that modifications thereto can be made without departure from the scope of the invention.

## Claims

1. A catheter (10) comprising:
a substantially elongated structure having a proximal portion (12), a distal portion (14), and exterior, and defining a main lumen (22) having a central axis and extending between the proximal and distal portions (12, 14), the main lumen (22) being in communication with the exterior of the elongated structure at least at said proximal and distal portions (12, 14);
**characterized in that** the main lumen has a continuous tubular inner surface, **in that** the catheter has a continuous outer surface, and **in that** the catheter also comprises a reinforcing wire (28, 30) having a cross section selected from oval and elliptical surrounding the main lumen (22) along at least a segment (20) of the substantially elongated structure, and
wherein the distal portion (14) of the catheter (10) exhibits a lateral stiffness represented by a deflection of greater than about 7.3°/cm-g measured by a Tinius-Olsen Stiffness Tester at 20°-30° of deflection.

2. A catheter (10) according to claim 1, wherein the substantially elongated structure comprises:
- an inner layer (26) having the continuous tubular inner surface defining the main lumen (22); and
- an outer layer (24) surrounding the inner layer (26) and having the continuous outer surface,
wherein the reinforcing wire (28, 30) is disposed between the inner and outer layers (26, 24).

3. A catheter (10) according to claim 1, wherein the substantially elongated structure comprises a single layer (36) in which the reinforcing wire (28, 30) is embedded, said single layer having the continuous tubular inner surface defining the main lumen and having the continuous outer surface.

4. A catheter (10) according to any of claims 1 to 3, wherein said reinforcing wire (28, 30) has a thickness (t) of about 0.01 mm to about 0.1 mm, and a width (w) of about 0.038 mm to 0.38 mm.

5. A catheter (10) according to any of claims 1-4, wherein the reinforcing wire (28, 30) is helically wound around the main lumen (22) along the segment (20).

6. A catheter (10) according to claim 5, wherein the helical winding is of varying pitches.

7. A catheter (10) according to any of claims 1-6, wherein the reinforcing wires (28, 30) comprise two wires (28, 30).

8. A catheter (10) according to claim 7, wherein the two wires (28, 30) are wound in opposite directions about the main lumen (22) along the segment (20).

9. A catheter (10) according to claim 7, wherein the two wires (28, 30) are interwoven.

10. A catheter (10) according to any of claims 1-9, wherein the distal portion (14) of the catheter (10) exhibits a critical bend diameter of no more than about 1.50 mm.

11. A catheter (10) according to any of claims 1-10, wherein the reinforcing wire (28, 30) has a thickness (t) of about 0.01 mm and a width (w) of about 0.038 mm.

12. A catheter (10) according to any of claims 1-10, wherein the reinforcing wire (28, 30) has a thickness (t) of about 0.03 mm and a width (w) of about 0.08 mm.

13. A catheter (10) according to any of claims 1-10, wherein the reinforcing wire (28, 30) has a thickness (t) of about 0.05 mm and a width (w) of about 0.08 mm.

14. A catheter (10) according to claims 1, 5, 6, 7, 8, or 9, wherein the wire cross-section is an oval cross-section.

15. A catheter (10) according to claims 1, 5, 6, 7, 8, or 9, wherein the wire cross-section is an elliptical cross-section.

## Patentansprüche

1. Katheter (10) mit
einer im Wesentlichen langgestreckten Struktur, die einen proximalen Abschnitt (12), einen distalen Abschnitt (14) und eine Außenseite hat und ein Hauptlumen (22) definiert, das eine zentrale Achse hat und sich zwischen den proximalen und distalen Abschnitten (12, 14) erstreckt, wobei das Hauptlumen (22) mindestens an den proximalen und distalen Abschnitten (12, 14) mit der Außenseite der langgestreckten Struktur in Verbindung steht,
**dadurch gekennzeichnet, dass** das Hauptlumen eine ununterbrochene rohrförmige Innenfläche hat, dass der Katheter eine ununterbrochene Außenfläche hat und dass der Katheter außerdem einen Verstärkungsdraht (28, 30) mit einem oval oder elliptisch gewählten Querschnitt aufweist, der das Hauptlumen (22) mindestens in einem Segment (20) der im Wesentlichen langgestreckten Struktur umgibt, und
bei dem der distale Abschnitt (14) des Katheters (10) eine Quersteifigkeit zeigt, dargestellt durch eine Ausbiegung von mehr als ungefähr 7,3 °/cm≅g, gemessen mit einem Tinius-Olsen-Steifigkeitsprüfgerät bei 20° bis 30° Ausbiegung.

2. Katheter (10) nach Anspruch 1, bei dem die im Wesentlichen langgestreckte Struktur Folgendes aufweist:
- eine innere Schicht (26), die die ununterbrochene rohrförmige Innenfläche aufweist, die das Hauptlumen (22) definiert, und
- eine äußere Schicht (24), die die Innenschicht (26) umgibt und die ununterbrochene Außenfläche aufweist,
wobei der Verstärkungsdraht (28, 30) zwischen den inneren und äußeren Schichten (26, 24) angeordnet ist.

3. Katheter (10) nach Anspruch 1, bei dem die im Wesentlichen langgestreckte Struktur eine einzelne Schicht (36) aufweist, in die der Verstärkungsdraht (28, 30) eingebettet ist, wobei die einzelne Schicht (36) die ununterbrochene rohrförmige Innenfläche, die das Hauptlumen definiert, und die ununterbrochene Außenfläche aufweist.

4. Katheter (10) nach einem der Ansprüche 1 bis 3, bei dem der Verstärkungsdraht (28, 30) eine Dicke (t) von ungefähr 0,01 mm bis ungefähr 0,1 mm und eine Breite (w) von ungefähr 0,038 mm bis 0,38 mm hat.

5. Katheter (10) nach einem der Ansprüche 1 bis 4, bei dem der Verstärkungsdraht (28, 30) entlang des Segments (20) schraubenförmig um das Hauptlumen (22) gewickelt ist.

6. Katheter (10) nach Anspruch 5, bei dem die schraubenförmige Wicklung wechselnde Ganghöhen hat.

7. Katheter (10) nach einem der Ansprüche 1 bis 6, bei dem die Verstärkungsdrähte (28, 30) zwei Drähte (28, 30) aufweisen.

8. Katheter (10) nach Anspruch 7, bei dem die zwei Drähte (28, 30) in entgegengesetzten Richtungen entlang des Segments (20) um das Hauptlumen (22) gewickelt sind.

9. Katheter (10) nach Anspruch 7, bei dem die zwei Drähte (28, 30) verflochten sind.

10. Katheter (10) nach einem der Ansprüche 1 bis 9, bei dem der distale Abschnitt (14) des Katheters (10) einen kritischen Biegedurchmesser von nicht mehr als ungefähr 1,50 mm zeigt.

11. Katheter (10) nach einem der Ansprüche 1 bis 10, bei dem der Verstärkungsdraht (28, 30) eine Dicke (t) von ungefähr 0,01 mm und eine Breite (w) von ungefähr 0,038 mm hat.

12. Katheter (10) nach einem der Ansprüche 1 bis 10, bei dem der Verstärkungsdraht (28, 30) eine Dicke (t) von ungefähr 0,03 mm und eine Breite (w) von ungefähr 0,08 mm hat.

13. Katheter (10) nach einem der Ansprüche 1 bis 10, bei dem der Verstärkungsdraht (28, 30) eine Dicke (t) von ungefähr 0,05 mm und eine Breite (w) von ungefähr 0,08 mm hat.

14. Katheter (10) nach Anspruch 1, 5, 6, 7, 8 oder 9, bei dem der Drahtquerschnitt ein ovaler Querschnitt ist.

15. Katheter (10) nach Anspruch 1, 5, 6, 7, 8 oder 9, bei dem der Drahtquerschnitt ein elliptischer Querschnitt ist.

## Revendications

1. Cathéter (10), comprenant :
une structure sensiblement allongée ayant une portion proximale (12), une portion distale (14), et un extérieur, et définissant une lumière principale (22) ayant un axe central et s'étendant entre les portions proximale et distale (12, 14), la lumière principale (22) étant en communication avec l'extérieur de la structure allongée au moins au niveau desdites portions proximale et distale (12, 14) ;
**caractérisé en ce que** la lumière principale a une surface intérieure tubulaire continue, **en ce que** le cathéter a une surface extérieure continue, et **en ce que** le cathéter comprend aussi un fil de renforcement (28, 30) ayant une section transversale choisie entre ovale et elliptique entourant la lumière principale (22) le long d'au moins un segment (20) de la structure sensiblement allongée, et
dans lequel la portion distale (14) du cathéter (10) montre une rigidité latérale représentée par une déviation supérieure à environ 7,3°/cm-g mesurée par un appareil à tester la rigidité Tinius-Olsen pour une déviation de 20°-30°.

2. Cathéter (10) selon la revendication 1, **caractérisé en ce que** la structure sensiblement allongée comprend :
- une couche intérieure (26) ayant la surface intérieure tubulaire continue définissant la lumière principale (22) ; et
- une couche extérieure (24) entourant la surface intérieure (26) et ayant la surface extérieure continue,
dans lequel le fil de renforcement (28, 30) est disposé entre les couches intérieure et extérieure (26, 24).

3. Cathéter (10) selon la revendication 1, dans lequel la structure sensiblement allongée comprend une simple couche (36) dans laquelle le fil de renforcement (28, 30) est incrusté, ladite simple couche ayant la surface intérieure tubulaire continue définissant la lumière principale et ayant la surface extérieure continue.

4. Cathéter (10) selon l'une quelconque des revendications 1 à 3, dans lequel ledit fil de renforcement (28, 30) a une épaisseur (t) d'environ 0,01 mm à environ 0,1 mm, et une largeur (w) d'environ 0,038 mm à 0,38 mm.

5. Cathéter (10) selon l'une quelconque des revendications 1 à 4, dans lequel le fil de renforcement (28, 30) est enroulé de manière hélicoïdale autour de la lumière principale (22) le long du segment (20).

6. Cathéter (10) selon la revendication 5, dans lequel l'enroulement hélicoïdal a des pas variables.

7. Cathéter (10) selon l'une quelconque des revendications 1 à 6, dans lequel les fils de renforcement (28, 30) comprennent deux fils (28, 30).

8. Cathéter (10) selon la revendication 7, dans lequel les deux fils (28, 30) sont enroulés dans des directions opposées autour de la lumière principale (22) le long du segment (20).

9. Cathéter (10) selon la revendication 7, dans lequel les deux fils (28, 30) sont entrelacés.

10. Cathéter (10) selon l'une quelconque des revendications 1 à 9, dans lequel la portion distale (14) du cathéter (10) montre un diamètre de flexion critique qui n'est pas supérieur à environ 1,50 mm.

11. Cathéter (10) selon l'une quelconque des revendications 1 à 10, dans lequel le fil de renforcement (28, 30) a une épaisseur (t) d'environ 0,01 mm et une largeur (w) d'environ 0,038 mm.

12. Cathéter (10) selon l'une quelconque des revendications 1 à 10, dans lequel le fil de renforcement (28, 30) a une épaisseur (t) d'environ 0,03 mm et une largeur (w) d'environ 0,08 mm.

13. Cathéter (10) selon l'une quelconque des revendications 1 à 10, dans lequel le fil de renforcement (28, 30) a une épaisseur (t) d'environ 0,05 mm et une largeur (w) d'environ 0,08 mm.

14. Cathéter (10) selon les revendications 1, 5, 6, 7, 8 ou 9, dans lequel la section transversale du fil est une section transversale ovale.

15. Cathéter (10) selon les revendications 1, 5, 6, 7, 8 ou 9, dans lequel la section transversale du fil est une section transversale elliptique.
